(19) Europäisches Patentamt
European Patent Office
Office européen des brevets

(11) **EP 2 390 345 A1**

(12) **DEMANDE DE BREVET EUROPEEN**

(43) Date de publication:
**30.11.2011 Bulletin 2011/48**

(51) Int Cl.:
*C12P 21/00* (2006.01)    *C07K 14/37* (2006.01)
*C12N 9/14* (2006.01)    *C12N 9/42* (2006.01)
*C12N 1/14* (2006.01)    *C12R 1/77* (2006.01)
*C12R 1/685* (2006.01)

(21) Numéro de dépôt: **11167963.5**

(22) Date de dépôt: **27.05.2011**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**

(30) Priorité: **27.05.2010 FR 1054122**

(71) Demandeurs:
• **Comptoir Agricole
67270 Hochfelden (FR)**
• **Universite De Strasbourg
67081 Strasbourg Cedex (FR)**

(72) Inventeurs:
• **Jeltsch, Jean-Marc
67120 MOLSHEIM (FR)**

• **Phalip, Vincent
67540 OSWALD (FR)**
• **Duranton, Jérôme
67000 STRASBOURG (FR)**
• **Tranier, Marie-Stéphane
67400 ILLKIRCH-GRAFFENSTADEN (FR)**
• **Misrahi, Audrey
59000 LILLE (FR)**
• **Falter, Cédric
67200 STRASBOURG (FR)**

(74) Mandataire: **Mouget-Goniot, Claire
Grosset-Fournier & Demachy
54, rue Saint-Lazare
75009 Paris (FR)**

(54) **Production de molecules d'interet par fermentation en milieu solide**

(57) Procédé de préparation de molécules d'intérêt par fermentation d'un substrat en phase solide.

EP 2 390 345 A1

**Description**

La présente invention a pour objet un procédé de préparation de molécules d'intérêt par fermentation d'un substrat en phase solide et leurs utilisations.

La biomasse représente une des ressources renouvelables les plus abondantes sur terre et certainement l'unes des moins coûteuses. Elle se définit comme étant « la fraction biodégradable des produits, déchets et résidus provenant de l'agriculture, y compris les substances végétales et animales, de la sylviculture et des industries connexes ainsi que la fraction biodégradable des déchets industriels et ménagés » (Article 29 de la loi 2005-781 du 13 juillet 2005). Les principales provenances de la biomasse sont, l'agriculture, la forêt, les milieux marins et aquatiques, les déchets verts, les industries et activités humaines traitant les matières d'origine vivante. Le développement de la valorisation de la biomasse constitue l'une des voies importantes pour participer à la maîtrise de la consommation des ressources non renouvelables et lutter contre les changements climatiques. Cette valorisation est croissante du fait de la multiplication et du renforcement, à l'échelle mondiale, des réglementations et des normes sanitaires et environnementales et du fait de la sensibilité de plus en plus forte des politiques et des citoyens au respect et à la préservation de l'environnement. Les filières de valorisation de la biomasse recouvrent trois grands secteurs, les biocarburants, les biocombustibles (chaleur, électricité) ainsi que celui des bioproduits ou biomatériaux. Parmi les technologies utilisées dans le cadre de la valorisation de la biomasse, la technologie de la fermentation en milieu solide (FMS) ouvre des perspectives prometteuses, notamment dans le secteur des biocarburants et des bioproduits.

Une fermentation en milieu solide (FMS) se définit comme une culture aérobie qui a lieu en l'absence d'eau libre dans le milieu (Hesseltine, (1987), International Biodeterioration 23:79-89; Pandey et al, (2001) Solid State Fermentation in Biotechnology - Fundamentals and Applications. Asiatech Publishers Inc.). Plus précisément, Saucedo-Castañeda (Contrôle du métabolisme de *Schwanniomyces castellii* cultivé sur support solide. Thèse, 212 pages 1991) propose la définition suivante : un procédé microbien où la culture se développe non seulement en surface mais aussi à l'intérieur d'une matrice poreuse solide et en l'absence d'écoulements de liquides. La matrice poreuse peut être constituée d'un substrat humide *per se* ou d'un support inerte capable d'absorber les nutriments qui se trouvent dissous en solution.

Bien que l'eau ne s'écoule pas, une phase hydrique est considérée ; elle correspond au film hydrophile qui recouvre chaque particule de la matrice solide. Cette eau joue un rôle essentiel puisqu'elle est impliquée dans le développement de la biomasse microbienne, les réactions métaboliques, les activités enzymatiques et le transport des gaz.

En FMS, la source de carbone est transformée de manière aérobie en biomasse microbienne, métabolites, $CO_2$ et $H_2O$. Ces transformations sont exothermiques ; elles donnent donc lieu à la libération de chaleur, dite chaleur métabolique. Une des caractéristiques d'une FMS est également son hétérogénéité microscopique.

De nombreux microorganismes se développent naturellement sur des supports solides biodégradables, à condition que suffisamment d'eau soit présente. Cette capacité a été utilisée par l'homme pour de nombreuses applications. Les premières fermentations en milieu solide ont été mises en oeuvre dans la fabrication du pain, qui date de 2600 ans avant l'an 1. Un autre procédé ancestral est celui de la fabrication du koji qui semble quasiment aussi ancien alors que la fabrication du tempeh et les techniques fromagères d'ensemencement des croûtes avec des champignons filamenteux correspondent également à des FMS.

La maîtrise de la fermentation en milieu solide a été essentiellement développée dans les pays d'Asie où cette technologie est à la base de nombreux produits alimentaires, comme la sauce soja, le tempeh ou encore le tofu. Depuis les années 80 une recherche active est menée également dans les pays occidentaux et porte notamment sur le contrôle de ce type de fermentation et sur la mise en évidence de son intérêt et de ses avantages pour diverses applications (Roussos et al, (1997), Proceedings of the 2nd International Symposium on Solid State Fermentation FMS-95, Montpellier, France in Kluwer Academic Publisher.).

La fermentation en milieu solide s'ajoute aux autres techniques de cultures que sont la fermentation en milieu liquide non agitée, assimilée à une fermentation de surface, la fermentation un milieu liquide agitée, assimilée à une fermentation submergée, la fermentation en milieu semi solide, où sont présents à la fois de l'eau libre et un solide.

La croissance de microorganismes sur milieux gélosés correspond à une fermentation en surface sur milieu solide ; elle présente quelques caractéristiques communes à une FMS, car elle a lieu en absence d'eau qui s'écoule, de manière aérobie et les microorganismes s'y développent de manière aérienne, mais contrairement à la FMS, le substrat n'est pas particulaire, ni poreux, ni aéré.

Ces différents procédés de culture (liquide, semi-solide, surface, solide) ont permis le développement de l'utilisation de microorganismes, soit pour les produire en grande quantité (par exemple pour fournir les boulangers et brasseurs en levures), soit pour produire des métabolites microbiens qui trouvent des applications dans de très nombreux domaines (par exemple pour produire la pénicilline et autres antibiotiques, ou les enzymes qui servent d'auxiliaires technologiques à des industries variées). Bien que la fermentation en milieu submergé (milieu nutritif liquide, parfaitement agité) soit la technique qui a été la plus développée dans les pays occidentaux et qu'elle soit encore la plus utilisée aujourd'hui, la fermentation en milieu solide est de plus en plus privilégiée. La technologie de la FMS, par opposition à celle de la fermentation en milieu liquide (FML), présente des avantages économiques, technologiques et environnementaux, offrant de ce fait une alternative prometteuse quant à la production d'enzymes ainsi que de métabolites divers. (production d'exopectinases par *Aspergillus niger az-*Godinez et al, (2001) Journal of Industrial Microbiology & Biotechnology, 26:271-275 ; production de phytases par *Aspergillus niger* Papagianni et al, (1999) Process Biochemistry, 35: 397-402.; production de pectinases par *Aspergillus niger* Patil et Dayanand, (2006) Bioresource Technology, 97: 2054-2058. ; production de protéase neutre par *Aspergillus oryzae* Sandhya et al, (2005) Process Biochemistry, 40: 2689-2694; enzymes fongiques Viniegra-Gonzalez et al, (2003) Biochemical Engineering Journal, 13:157-167.).

Les microorganismes (bactéries, levures, champignons) produisent un grand nombre d'enzymes. Ces enzymes, nécessaires à leur croissance, sont regroupées selon différentes familles (cellulases, hémicellulases, amylases, phosphatases, pectinases, protéases, lipases, etc.) et trouvent des applications dans de nombreux domaines industriels.

Les amylases, enzymes impliquées dans la dégradation de l'amidon sont ainsi utilisées pour fabriquer du sirop de glucose ou encore du sirop de maltose. Les applications du sirop de glucose sont les confiseries (conserves de fruits, fruits confits, confitures, compotes), la brasserie, la fabrication de liqueurs, la fabrication de boissons non alcoolisées. Le sirop de maltose est utilisé plus précisément en brasserie, panification, fabrication des boissons, crèmes glacées et dans l'industrie pharmaceutique (surtout en remplacement du glucose pour les diabétiques). La fabrication de sirop de fructose est un autre débouché, qui s'est beaucoup développé grâce au pouvoir sucrant du fructose supérieur à celui du saccharose et celui du glucose. Les amylases sont également incorporées dans les détergents pour participer à l'élimination des tâches de couleur, pour la production des biocarburants de première génération.

Les phosphatases, famille d'enzymes très variée qui catalysent l'hydrolyse des liaisons phosphomonoesters, sont elles utilisées en alimentation humaine et animale. Au sein des différentes classes de phosphatases, il existe des enzymes capables d'hydrolyser une ou plusieurs des liaisons phosphomonoesters de l'acide phytique (myo-inositol 1,2,3,4,5,6-hexa*kis*phosphate), entraînant ainsi la libération de phosphates inorganiques et de *myo*-inositol phosphates plus ou moins phosphatés. Cette activité est nommée activité phytase. L'ajout de phytases dans la formulation des rations alimentaires des animaux monogastriques d'élevage permet un double effet sur l'environnement : une diminution de l'ajout de phosphate inorganique d'une part et une diminution de la quantité de phytate rejeté dans l'environnement d'autre part. Elles sont utilisées en nutrition humaine pour éviter les déficiences minérales dues à l'acide phytique dans les zones où les céréales sont la principale nourriture des populations.

Les protéases qui constituent l'un des trois plus grands groupes d'enzymes industriels trouvent des applications dans l'industrie des détergents, du textile, du cuir, de l'alimentaire ou dans l'industrie pharmaceutique. Les protéases sont, par exemple, intégrées dans les lessives afin d'éliminer les taches à base de protéines. Dans l'industrie textile, les protéases sont utilisées pour modifier la surface des fibres de laine et de soie dans le but d'améliorer le lustrage et la douceur des tissus. Dans l'industrie du cuir, le processus d'épilation des peaux fait aussi intervenir l'activité biologique des protéases.

Les lipases font partie de la famille des hydrolases qui agissent sur les liaisons esters carboxyliques. Le rôle physiologique des lipases est d'hydrolyser les triglycérides en diglycérides, monoglycérides, acides gras et de glycérol. Cette famille d'enzyme constitue un des groupes de biocatalyseurs qui trouve de nombreuses applications biotechnologiques dans des domaines aussi variés que ceux de l'industrie de l'alimentation, des détergents, pharmaceutique, du cuir, du textile, du papier ou de la cosmétique.

Les enzymes qui interviennent dans la dégradation complète de la cellulose sont : l'endo- β-1,4-D-glucanase, l'exo-β-1,4-D-glucanase (encore appelée cellobiohydrolase) et la β-glucosidase ; les enzymes qui hydrolysent les pectines sont les pectate lyases, les pectinases, les pectines estérases, les polygalacturonases ; toutes ces enzymes capables de dégrader les polymères des parois végétales trouvent de multiples applications dans des secteurs variés. Par exemple, les pectinases sont utilisées dans les industries des jus de fruits, pour les clarifier et faciliter leur filtration. Les glucanases (cellulases et hemicellulases) présentent des marchés diversifiés. Elles sont utilisées dans l'industrie du textile pour l'attendrissement du coton ou pour la finition du denim, dans le domaine des détergents pour fabriquer des poudres de lavages qui respectent les couleurs, facilitent le nettoyage et évitent les phénomènes de redéposition des salissures. Dans l'industrie de la pulpe de papier, les xylanases sont utilisées pour réduire l'utilisation de chlore lors de l'étape de blanchiment. Les hémicellulases sont aussi utilisées dans le domaine de la panification où elles offrent de nombreux avantages tels que l'augmentation des volumes spécifiques des pains ou du gain de tolérance à la fermentation. Des cellulases pour ruminants, permettant d'améliorer la digestibilité de leur ration en fourrage, pourraient présenter un intérêt. Les cellulases sont également utilisées dans le secteur de l'énergie pour la conversion des déchets cellulosiques en éthanol.

Toutes ces molécules d'intérêts (enzymes et métabolites) sont individuellement disponibles dans le commerce sous forme purifiée, mais ont l'inconvénient d'être relativement onéreuses.

Il existe donc un besoin de disposer d'un procédé permettant de produire ces diverses molécules d'intérêt, en cocktail ou purifiée, à coût réduit et avec un bon rendement.

La présente invention vise à satisfaire ce besoin.

La présente invention concerne un procédé de préparation de molécules d'intérêt par fermentation d'un substrat en phase solide comprenant les étapes suivantes :

a) prendre un substrat,

b) réduire éventuellement la taille dudit substrat de manière à obtenir des particules dont 100 % ont un diamètre moyen inférieur à 1 mm,

c) stériliser le substrat à sec,

d) inoculer sous agitation le substrat obtenu à l'étape c) avec un microorganisme en suspension à raison de 1 x $10^5$ à 1 x $10^7$ spores/gramme de substrat, avantageusement 2,5 x $10^5$ spores/gramme de substrat, et de manière à avoir une $a_w$ comprise entre 0,950 et 0,999, avantageusement comprise entre 0,980 et 0,995,

e) laisser fermenter à l'état solide dans un réacteur sans agitation pendant 2 à 8 jours, avantageusement 3 à 6 jours, à une température comprise entre 18 et 30 °C sous pression atmosphérique,

f) stériliser le substrat fermenté obtenu à l'étape e),

g) reprendre le substrat obtenu à l'étape f) par un volume d'eau représentant 0,5 à 5 fois la masse de substrat,

h) recueillir le liquide de lavage,

i) extraire les molécules d'intérêt à partir du liquide recueilli à l'étape h), et

j) optionnellement, sécher les molécules d'intérêt recueillies à l'étape i)

Au sens de la présente invention on entend par « molécules d'intérêt », tout produit issu du métabolisme microbien, en particulier,

- des enzymes, notamment choisies dans le groupe comprenant les cellulases, les xylanases, les α-amylases, les arabinoxylanases, les α-et β-glucosidases, les fucosidases, les protéases, les phytases, les phosphohydrolases, les phosphatases, les estérases, les glucosaminidases, les α-et β-galactosidases, les α-mannosidases et les lipases,
- des vitamines,
- des substances à activité thérapeutiques comme par exemple les antibiotiques,
- des pigments,
- des polysaccharides
- des lipides.
- des antioxydants

Au sens de la présente invention on entend par « substrat » la biomasse végétale, les matières premières agricoles, les sous-produits de l'industrie alimentaire et les boues d'épuration, notamment,

- les céréales comme par exemple le maïs, le blé, l'orge et le riz,
- le son de blé,
- la paille,
- les rafles de maïs,
- le bois de résineux ou de feuillu,
- les déchets végétaux, notamment les herbes et les feuilles,
- les déchets d'amidonnerie,
- la pulpe de betterave,
- le papier,
- la bagasse de canne à sucre,
- les résidus de papeterie (notamment les boues).

Au sens de la présente invention on entend par « microorganismes », les bactéries, et les champignons filamenteux ou non, en particulier les levures. A titre d'exemple de bactérie, on peut citer notamment *Bacillus* et *Escherichia coli* et à titre d'exemple de champignons *Fusarium, Trichoderma, Aspergillus, Penicillium* et *Rhizopus,* en particulier *Fusarium venenatum, Fusarium graminearum, Fusarium avenaceum, Fusarium tricinctum, Fusarium sambucinum, Phoma exigua, Alternaria alternata, Epicoccum nigrum, Sclerotinia sclerotiorum,* obtenus à partir de plants de houblon en dépérissement et isolés sur milieu PDA selon la technique décrite par Hatsch et al, (2002), (Development of a bipartite method for Fusarium identification based on cellobiohydrolase-C : CAPS and Western blot analysis Fems Microbiology letters 213 :245-249), *Trichoderma harzianum* (déposé sous le no DSM 63059 à la Collection allemande de microorganismes), *Penicillium camembertii* prélevé sur du camembert, , *Saccharomyces cerevisiae.*

Dans le cadre de la présente invention la stérilisation à l'étape c) peut être réalisée par toutes techniques connues de l'homme du métier, notamment par traitement thermique (notamment avec plaques électriques, autoclave...) ou par irradiation avec un rayonnement alpha, béta (électron), gamma ou X. L'irradiation du substrat à l'étape c) induit l'ionisation de la biomasse dans le but de la stériliser ainsi que de la préparer à la croissance d'un champignon. Dans un mode de réalisation avantageux de l'invention, la stérilisation à l'étape f) est réalisée antérieurement à l'extraction des molécules d'intérêt. Cette utilisation pour le post traitement de la biomasse fermentée, a pour objectif de détruire, par des rayonnements ionisants, le champignon et ses spores tout en conservant l'intégrité des molécules d'intérêt. Les conditions d'irradiation seront adaptées à chaque substrat et ces adaptations sont à la portée de l'homme du métier. Les conditions d'irradiation de l'étape f) sont adaptées à chaque champignon ainsi qu'à chaque famille de molécules d'intérêt.

Dans un mode de réalisation avantageux de l'invention, l'une au moins des étapes c) et f) est réalisée par irradiation, avantageusement les deux étapes sont réalisées par irradiation.

L'étape e) peut être réalisée dans tout type de réacteur connu de l'homme du métier.

L'extraction et le séchage des molécules respectivement à l'étape i) et à l'étape j quand elle existe à la suite de l'étape h) peuvent également être réalisés par n'importe quelles techniques connues de l'homme du métier, notamment par pressage du liquide suivi d'un séchage par atomisation.

Dans un autre mode de réalisation avantageux de l'invention, l'étape d) est réalisée en milieu aqueux, en présence ou non d'un tampon ; à titre d'exemple le milieu peut-être de l'eau à pH 6, la quantité d'eau représentant de 0,1 à 2 fois la masse du substrat, avantageusement 1 fois.

Dans un mode de réalisation particulièrement avantageux de l'invention, les microorganismes utilisés sont *Fusarium venenatum* déposé le 5 septembre 2001 sous le no IMI 386918 ainsi *qu'Aspergillus niger* déposé le 5 mai 2011 sous le no IMI 500512 à CABI GRC (CAB International Genetic Resource Collection Nosworthy Way Wallingford Oxfordshire OX10 8DE Grande-Bretagne).

Dans un autre mode de réalisation particulièrement avantageux de l'invention les molécules d'intérêts obtenues sont choisies dans le groupe comprenant les cellulases, les xylanases, les α-amylases, les arabinoxylanases, les α-et β-glucosidases, les fucosidases, les protéases, les phytases, les phosphohydrolases, les phosphatases, les estérases, les glucosaminidases, les α-et β-galactosidases, les α-mannosidases et les lipases.

Dans encore un autre mode de réalisation particulièrement avantageux de l'invention, le substrat est le son de blé, le microorganisme est *Fusarium venenatum* déposé sous le no : IMI 386918 ainsi *qu'Aspergillus niger* déposé sous le no IMI 500512 à CABI GRC (CAB International Genetic Resource Collection Nosworthy Way Wallingford Oxfordshire OX10 8DE Grande-Bretagne).

Conformément à l'invention, les molécules d'intérêt obtenues par le procédé selon l'invention peuvent être utilisées pour l'alimentation humaine et l'alimentation animale, dans l'industrie du papier, l'industrie des détergents et l'industrie des textiles, pour le traitement et la valorisation des déchets végétaux, en particulier la production du bioéthanol et pour la bio protection des cultures.

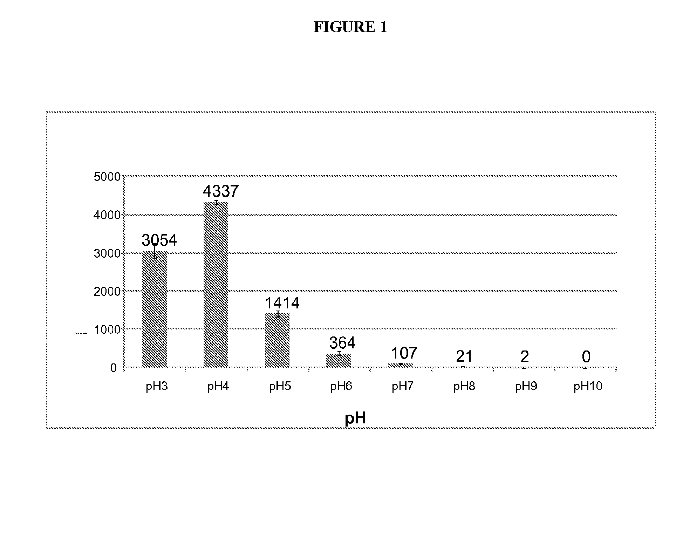

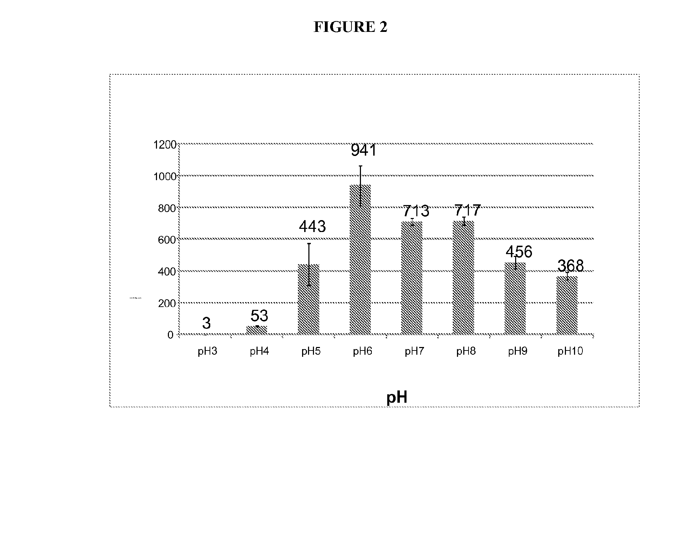

L'invention sera mieux comprise à l'aide des exemples 1 à 6 et des figures 1 et 2 qui suivent.

**La figure 1** représente l'activité xylanolytique en fonction du pH pour *Aspergillus niger* telle que mesurée selon l'exemple 4.

**La figure 2** représente l'activité xylanolytique en fonction du pH pour *Fusarium venenatum* telle que mesurée selon l'exemple 4.

**Exemple 1 MATERIEL ET METHODES**

**1.1 Matériels**

**1.1.1** *Substrats de fermentation en milieu solide (FMS)*

Les substrats utilisés sont choisis parmi : maïs, blé, orge, riz, son de blé, paille, bois (résineux et feuillus), déchets végétaux (herbes et feuilles...), pulpe de betterave, papier, bagasse de canne à sucre, boues de papeterie.

**1.1.1.1** *Caractérisation de la matière première*

Activité de l'eau

L'activité de l'eau des préparations de substrats est déterminée en utilisant un appareil de mesure ($a_w$ mètre) de chez Fast-Lab (GBX) placé dans une pièce climatisée à 23°C. L'échantillon est introduit dans une coupelle sèche et immédiatement placé dans la chambre de mesure. L'équipement est préalablement étalonné avec une solution saline de NaCl.

Matière sèche

La masse sèche des substrats solides est déterminée après séchage dans une étuve à 130°C pendant deux jours (suivi d'un retour à température ambiante dans un dessiccateur). Leur teneur en eau est déterminée selon la formule.

$$\text{Teneur en eau} = 100*((\text{masse humide} - \text{masse sèche})/ \text{masse humide})$$

Flore initiale ou résiduelle

En routine, les vérifications de la flore présente dans le milieu sont effectuées de la manière suivante. Un fragment de substrat est inoculé sous hotte, à l'aide d'une spatule préalablement stérilisée :

■ d'une part dans du LB (Lennox Broth) liquide autoclavé (10 g/L de tryptone ; 5 g/L d'extrait de levure ; 5 g/L de chlorure de sodium),
■ d'autre part sur du PDA (Potato Dextrose Agar : 4 g/L extrait de pomme de terre ; 20 g/L de dextrose ; 15 g/L d'agar).

Le milieu LB est mis à incuber à 37°C sous agitation (300 rpm) et l'absence de trouble est vérifiée après 24 et 48h. En cas de trouble, un étalement est observé au microscope et/ou un prélèvement étalé sur du LB agar. Ce test est pratiqué sur les substrats initiaux et sur les matrices fermentées. Le milieu PDA est mis à incuber à 24°C pour vérifier l'absence ou la présence de développement fongique. Ce test est mené sur les substrats initiaux uniquement.

### 1.1.2 *Matériel microbiologique*

#### 1.1.2.1 *Origine des souches*

Le Tableau 1 synthétise la provenance des souches employées dans les exemples.

**Tableau 1 : Origine des souches utilisées.**

| Souche | Provenance |
|---|---|
| Fusarium venenatum | |
| Fusarium graminearum | |
| Fusarium avenaceum | |
| Fusarium tricinctum | Obtenues à partir de plants de houblon en dépérissement et isolés sur milieu PDA (Hatsch et al, 2002) |
| Fusarium sambucinum | |
| Phoma exigua | |
| Alternaria alternata | |
| Epicoccum nigrum | |
| Sclerotinia sclerotiorum | |
| Trichoderma harzianum | DSM (Deutsche Sammlung für Microorganisms) |
| Penicillium camembertii | Prélevée sur du camembert |
| Escherichia coli | |
| Saccharomyces cerevisiae | Souches utilisées en biologie moléculaire au laboratoire (Invitrogen SARL) |

#### 1.1.2.2 *Milieux pour la production de biomasse et de conservation des souches*

Le milieu PDA est utilisé pour la croissance des champignons en culture de surface et leur conservation. Le milieu LB Agar (milieu LB avec 15 g/L d'agar) est utilisé pour la croissance des bactéries. Le milieu YPG (10 g/L d'extrait de levures ; 20 g/L de peptone ; 20 g/L de glucose) est utilisé pour la croissance des levures et des champignons en phase liquide.

#### 1.1.2.3 *Milieu standard de sporulation de Fusarium*

Le milieu SNA (Synthetischer Nährstoffärmer Agar, Tableau 2) est utilisé pour la sporulation de *Fusarium* (selon Stack, (1989), A Comparaison of the Inoculum Potential of Ascospores and Conidia of Giberella zeae. Canadian Journal of Plant Pathology, 11 :137-142). Le milieu est inoculé avec un fragment de mycélium et incubé à 24°C. Pour récolter les spores produites sur SNA, la surface de la culture est aspergée délicatement d'eau ultrapure (Millipore®) stérile avec une pipette de 10mL. La boîte est doucement remuée. Puis le liquide chargé en spores est aspiré.

**Tableau 2 : Ingrédients du milieu SNA et quantités pour 1L de culture.**

| composé | quantité |
|---|---|
| $KH_2PO_4$ | 1 g |
| $KNO_3$ | 1 g |
| $MgSO_4.7H_2O$ | 0,5 g |
| KCl | 0,5 g |
| Glucose | 0,2 g |
| Saccharose | 0,2 g |
| Agar | 15 g |
| $Na_2B_4O_7.10H_2O$ | 0,04 mg |
| $MnSO_4.H_2O$ | 0,8 mg |

Les spores de *Fusarium graminearum* utilisées dans ces travaux proviennent de récoltes issues de ce milieu, ainsi que les premières spores de *F. venenatum* employées. Puis le milieu développé dans ces travaux à base de son de blé a été utilisé comme source de nouvelles spores, qu'il permet d'obtenir en plus grande quantité (voir paragraphe 1.1.2.4).

### 1.1.2.4 *Récolte de spores produites par FMS*

Pour récolter les spores produites par FMS, la matrice fermentée est mise en suspension dans de l'eau ultrapure. Après une vive agitation, la suspension est mise à infuser à 4°C sous agitation pendant une heure. Le surnageant est pipeté stérilement et introduit dans un Falcon, pour être centrifugé à 10000g. Les spores culottées sont lavées plusieurs fois à l'eau ultrapure puis réunies dans un faible volume. Ce lot est nommé stock FMS.
Les spores sont dénombrées au microscope en utilisant une cellule de Neubauer. Les spores sont conservées à -20°C en aliquotes de concentration connue, dans de l'eau ou de l'eau glycérolée à 20%.

### 1.1.2.5 *Evaluation de la biomasse*

#### 1.1.2.5.1 Mesures directes

Mesure de matière sèche de mycélium

Le mycélium total d'une fermentation en milieu liquide est récupéré sous forme de culot suite à la centrifugation du milieu de culture à 10000g pendant 10 minutes à 10°C. Alternativement, le mycélium est récupéré sur le filtre d'une unité de filtration à vide sur 0,22$\mu$m (Stericup®, Millipore). Le mycélium est ensuite déposé sur un filtre en microfibres de verre sec pré taré et mis à sécher dans une étuve à 90°C pendant 48 heures. Le mycélium sec sur filtre est pesé et la quantité de mycélium sec produite est déduite. Elle est ensuite rapportée à une masse de mycélium sec par litre de milieu de culture.

Mesure de surface de colonisation du mycélium

Suite à une inoculation par spot d'une goutte de suspension de spores ou d'un fragment de mycélium sur le substrat-support de culture solide, le champignon se développe (après germination des spores, le cas échéant) en émettant des hyphes dans toutes les directions. Les hyphes se ramifient et une croissance exponentielle est obtenue. Ce développement dans toutes les directions aboutit à une croissance globalement concentrique, visible à l'oeil nu, qui peut se caractériser par un diamètre moyen à un temps donné. Ce diamètre moyen correspond à la moyenne de trois mesures de la taille de la colonie, prises en passant par son centre. La taille du mycélium est ensuite déterminée en l'assimilant à un disque, dont la surface, dite surface de colonisation, se calcule de la manière suivante : surface de colonisation = $\pi$ x (diamètre moyen/2)$^2$. Le suivi de l'évolution de la taille du mycélium qui se développe à la surface du milieu de culture permet de calculer ensuite la vitesse de colonisation du milieu par le champignon.

Dénombrement des spores

Pour dénombrer les spores d'une matrice solide, une masse connue (m) de celle-ci est reprise dans un tampon. Après agitation, un prélèvement de la suspension est dénombré avec la cellule de Neubauer selon l'exemple 1.1.2.3). L'indice de sporulation (quantité de spores/ g de poids sec de la matrice fermentée) est calculé selon :

[spores/$\mu$L] x volume de l'extraction / masse de matrice sèche.

**1.1.2.5.2 Mesures indirectes**

L'évaluation de la croissance fongique est pratiquée dans les expériences de FMS en se basant sur l'évolution de la concentration en protéines (mesurées au Bradford), la présence de NADH oxydase et l'évolution de la quantité de *N*-acétyl-D-glucosamine libre.
Des dosages immunologiques d'un constituant fongique pratiqués par ELISA et des suivis par immuno dot blot sont employés pour participer à des évaluations indirectes de la biomasse fongique.

**1.2 Méthodes**

**1.2.1 Préparation des milieux**

**1.2.1.1** *Réduction de taille*

La taille réduction de la taille des substrats se fait à l'aide d'un broyeur à marteaux Retsch SK100 Confort, avec une grille de trous coniques de 1mm.
Un broyeur à marteaux est constitué d'une chambre à l'intérieur de laquelle trois marteaux formant un Y tournent et percutent les substrats qui sont introduits dans cette chambre *via* une trémie. L'espace entre les marteaux et les parois de la chambre est étroit, ainsi ces chocs provoquent la rupture des substrats, et la réduction des particules formées.

**1.2.1.2** *Traitements des substrats par autoclavage ou irradiation*

Autoclavage

Les substrats sont ensachés. Le barème du traitement autoclave est 121°C pendant 20 minutes à une pression de 0,3 MPa (3 bars)

Irradiation (ionisation).

L'ionisation est un procédé qui utilise les rayonnements corpusculaires (faisceaux d'électrons) pour préserver ou stériliser un produit, ou encore améliorer ou modifier ses caractéristiques.
Les substrats ensachés sont irradiés dans les conditions suivantes :

- sous air
- à la dose de 100 kGy, préférentiellement de 5 kGy à 100 kGy
- par faisceaux d'électron de 2,2 meV d'énergie
- à un débit dose de 1,5 kGy/s
- à température ambiante

**1.2.1.3** *Formulation des milieux*

L'humidification des substrats a lieu après le traitement par autoclavage ou par irradiation de ceux-ci. Le tampon stérile utilisé est le Tris-Hcl 100 mM, pH=10.5 L'humidification des substrats est appliquée en visant un rapport volume de solution/masse de substrat broyé prédéterminé à l'aide d'un appareil de mesure $a_w$mètre ($a_w$=0.99). Les milieux sont dans chaque cas préparés de telle sorte que toute la solution d'humidification soit absorbée, afin qu'il n'y ait pas d'eau libre.

**1.2.1.4** *Conditions de culture de la FMS*

Les substrats sont introduits dans le fermenteur après avoir été humidifiés à l'aide du tampon Tris-Hcl 100 mM pH=10,5 et ensemencés à 2,5x10$^5$ spores/gramme de substrat. La FMS est réalisée sous pression atmosphérique, la température du fermenteur est maintenue à 23°C, l'humidité à 99% et l'oxygène est renouvelé en continu. Le processus de fermentation se fait sur une durée de 4 jours sans agitation. Le temps zéro des cultures correspond au moment où les spores sont inoculées sur le substrat solide.

*1.2.1.5 Extraction et formulation des molécules issues du processus de FMS*

- Les matrices fermentées sont extraites du fermenteur après 4 jours d'incubation puis traitées par irradiation dans les conditions suivantes
- sous air
- à la dose de 5 kGy à 100 kGy
- par faisceaux d'électron de 2,2 meV d'énergie
- à un débit dose de 1,5 kGy/s
- à température ambiante

Les matrices fermentées irradiées sont reprises dans un volume d'eau équivalent à 5 fois leur poids. Après homogénéisation, les matrices sont pressées à l'aide d'un pressoir mécanique. Les jus récupérés, contenant les molécules d'intérêt sont séchés par lyophilisation ou atomisation. Les molécules d'intérêt présentes dans les poudres issues du séchage sont testées selon des protocoles standards connus de l'homme du métier.

**Exemple 2 : Cas du son de blé et de *Fusarium venenatum :* production de xylanases**

Le substrat utilisé est du son de blé en provenance de moulins. L'activité de l'eau du son de blé est déterminée en utilisant un appareil de mesure ($a_w$mètre) de chez Fast-Lab (GBX) placé dans une pièce climatisée à 25°C. La valeur de l'Aw obtenu sur ledit substrat est de 0,385. La teneur en humidité obtenue par la détermination de la masse sèche du substrat est de 8,8%.

Le microorganisme utilisé est *Fusarium venenatum,* souche déposé le 5 septembre 2001 sous le no : IMI 386918 à CABI GRC (CAB International Genetic Resource Collection Nosworthy Way Wallingford Oxfordshire OX10 8DE Grande-Bretagne) et identifié le 24 octobre 2001. Les spores du champignon nécessaire à la réalisation de la FMS ont été produites selon la procédure décrite dans l'exemple 1 (1.1.2.3, 1.1.2.4).

1 kg de son de blé ensaché a été autoclavé selon les conditions suivantes :

- température : 121°C
- temps : 20 min
- pression : 0,3 MPa (3 bars)

Le son de blé prétraité est alors extrait du sachet, humidifié à l'aide de 1.2 litre de tampon Tris-Hcl 100 mM pH=10 et ensemencés à $1 \times 10^6$ spores/gramme de substrat. Le substrat humidifié et inoculé est alors introduit dans le fermenteur. La FMS est réalisée sous pression atmosphérique, la température du fermenteur est maintenue à 25°C, l'humidité à 99% et l'oxygène est renouvelé en continu. Le processus de fermentation se fait sur une durée de 4 jours sans agitation. Le temps zéro des cultures correspond au moment où les spores sont inoculées sur le substrat solide.

- La matrice fermentée est extraite du fermenteur après 4 jours d'incubation puis traitée par irradiation dans les conditions suivantes :
- sous air
- à la dose de 10 kGy
- par faisceaux d'électron de 2,2 meV d'énergie
- à un débit dose de 1,5 kGy/s
- à température ambiante

La biomasse fermentée traitée est reprise dans 5 litres d'eau. Après homogénéisation, la matrice est pressée à l'aide d'un pressoir mécanique. Le jus récupéré contenant la molécule d'intérêt est filtré puis séché par atomisation. Les xylanases présentes dans la poudre issue du séchage par atomisation sont testées selon le protocole décrit ci-dessous.

100 mg de poudre issue de l'atomisation est dissous dans 10 ml d'eau (échantillon A). L'activité xylanolytique présente dans la poudre est déterminée par la méthode décrite par Bailey et al. (Interlaboratory testing of methods for assay of xylanase activity Journal of Biotechnology, Volume 23, Issue 3, Pages 257-270). Une solution 1% (p/v) de xylan de bouleau (Sigma USA) préparée dans du tampon phosphate 100 mM, pH 6 est utilisée comme substrat. 50 μl de la solution A est introduit dans une cuvette contenant 950 μl de substrat. Le mélange substrat échantillon A est incubé à 37 °C pendant 60 minutes. Et un prélèvement de 100μl est effectué toutes les 10 minutes. La réaction d'hydrolyse est stoppée par addition de 800 μl de DNS (dinitrosalicylic acid). Le mélange est chauffé pendant 10 minutes à 100°C et l'absorbance résultant de la réaction d'hydrolyse est mesurée à 550 nm. L'activité est évaluée en dosant les sucres réducteurs libérés par rapport à une courbe étalon réalisée avec une solution standard de xylose. Une unité de xylanase est définie comme le nombre de μmol d'extrémités réductrices libérées par min et par ml d'échantillon A.

L'activité xylanolytique présente dans l'échantillon A obtenue par notre procédé de fabrication est égale à 9,4 U/ ml d'échantillon A, soit 940 U/ g de poudre.

**Exemple 3 : Cas du son de blé et *d'Aspergillus niger* : production de xylanases**

Le substrat utilisé est du son de blé en provenance de moulins. L'activité de l'eau du son de blé est déterminée en utilisant un appareil de mesure (a$_w$mètre) de chez Fast-Lab (GBX) placé dans une pièce climatisée à 25°C. La valeur de l'Aw obtenue dudit substrat est de 0,365. La teneur en humidité obtenue par la détermination de la masse sèche du substrat est de 8,8%.

Le microorganisme utilisé est *Aspergillus niger,* souche déposée le 05 mai 2011 sous le no IMI 500512 à CABI GRC (CAB International Genetic Resource Collection Nosworthy Way Wallingford Oxfordshire OX10 8DE Grande-Bretagne). Les spores du champignon nécessaires à la réalisation de la FMS ont été produites selon la procédure décrite dans l'exemple 1 (1.1.2.3, 1.1.2.4).

1 kg de son de blé ensaché a été irradié selon les conditions suivantes :

- sous air
- à la dose de 30 kGy
- par faisceaux d'électron de 2,2 meV d'énergie
- à un débit dose de 1,5 kGy/s
- à température ambiante

Le son de blé prétraité est alors extrait du sachet, humidifié à l'aide de 1,2 litres de tampon Tris-Hcl 100 mM pH=10 et ensemencé à 1x10$^6$ spores/gramme de substrat. Le substrat humidifié et inoculé est alors introduit dans le fermenteur. La FMS est réalisée sous pression atmosphérique, la température du fermenteur est maintenue à 25°C, l'humidité à 99% et l'oxygène est renouvelé en continu. Le processus de fermentation se fait sur une durée de 4 jours sans agitation. Le temps zéro des cultures correspond au moment où les spores sont inoculées sur le substrat solide.

La matrice fermentée est extraite du fermenteur après 4 jours d'incubation puis traitée par irradiation dans les conditions suivantes :

- sous air
- à la dose de 20 kGy
- par faisceaux d'électron de 2,2 meV d'énergie
- à un débit dose de 1,5 kGy/s
- à température ambiante

La biomasse fermentée traitée est reprise dans 5 litres d'eau. Après homogénéisation, la matrice est pressée à l'aide d'un pressoir mécanique. Le jus récupéré contenant les molécules d'intérêt est filtré puis séché par atomisation. Les xylanases présentes dans la poudre issue du séchage par atomisation sont testées selon le protocole décrit ci-dessous.

100 mg de poudre issue de l'atomisation sont dissous dans 10 ml d'eau (échantillon A). L'activité xylanolytique présente dans la poudre est déterminée par la méthode décrite dans l'exemple 2.

L'activité xylanolytique présente dans l'échantillon A obtenue par notre procédé de fabrication est égale à 14,3 U/ ml d'échantillon A, soit 1413 U/ g de poudre.

**Exemple 4 : Activité xylanolytique des enzymes produites par *Fusarium venenatum* et *Aspergillus niger* en fonction du pH.**

Les microorganismes utilisés sont *Fusarium venenatum* déposé le 5 septembre 2001 sous le no IMI 386918 ainsi qu'*Aspergillus niger* déposé le 5 mai 2011 sous le no IMI 500512 à CABI GRC (CAB International Genetic Resource Collection Nosworthy Way Wallingford Oxfordshire OX10 8DE Grande-Bretagne).

Le dosage des activités enzymatiques ont été réalisés en triplicata selon la procédure décrite dans l'exemple 2.

Les figures 1 et 2 montrent que l'activité enzymatique est fortement modulée par le pH. Ces propriétés permettent la mise en adéquation du choix des enzymes et donc du champignon en fonction de l'application industrielle visée (cf. exemple 5 et 6).

**Exemple 5 : Cas du son de blé et de *Fusarium venenatum*.**

**Test en application : bio-blanchiment de pâte à papier Kraft d'Eucalyptus**

Les essais ont été réalisé au Centre Technique du Papier de Grenoble (Domaine Universitaire -BP 251 - 38044 GRENOBLE - Cedex 9 - France).

**1. Matériels et méthodes**

**1.1 Enzymes :**

Les enzymes utilisés ont été produits comme indiqué dans l'exemple 2.

**1.2 Echantillons de pâtes Kraft d'Eucalyptus**

Les essais de bio-blanchiment ont été menés sur des pâtes Kraft *d'Eucalyptus globulus* industrielles. La séquence confidentielle de blanchiment industrielle de l'usine est OO Q PoP.
Le traitement enzymatique sera judicieusement placé à la suite des stades O.
Des échantillons de pâte Kraft écrue (Ref. PV09.341.P) et de pâte délignifiée (après OO, Ref PV09.341.BP) ont donc été fournis par un industriel papetier. Les résultats des données fournisseurs et des mesures sont présentés dans le Tableau ci-dessous :

Tableau 3 : Caractéristiques des pâtes Kraft d'*Eucalyptus Globulus*

| Echantillon | Ref. | Siccité[1] (%) | IK[2] | Blancheur[2] (%ISO) | L, a, b **[4] | Viscosité[5] (ml/g) | [HexA][6] ($\mu$mol/g) |
|---|---|---|---|---|---|---|---|
| Pâte Kraft écrue | PV09.341.P | 25,1 | 15,9 $\pm$ 0,1* | 3,.4 $\pm$ 0,1* | 77,7 2,5 16,5 | 1188 $\pm$ 10* | 91,1** |
| Pâte délignifiée (après stade OO) | PV09.341.BP | 25,6 | 8,6 $\pm$ 0,1* | 63 $\pm$ 0,1* | 90,3 0,07 12,9 | 944 $\pm$ 10* | 81,8** |

*données du fournisseur
**mesuré au laboratoire du CTP
[1]siccité (NF EN 20287), [2]Indice Kappa (IK) (NF ISO 302), [3]Indice de Blanc (Blancheur R457 UV C, NFISO 2470), [4]facteurs L*, a*, b* (ISO 5631), [5] viscosité (ISO 5351), [6] Acides hexenuroniques (méthode spectroscopique, Chai *et al.* 2001)

**1.3 Méthodes**

L'étude de bio-blanchiment a été conduite selon la séquence X Q PoP, sur 50g eq. sec de pâte Kraft délignifiée (OO) diluée dans l'eau du robinet à une consistance de Cp=10% (w/w) avec :

- X : stade enzymatique: 50°C, pH6 (ajusté avec $H_2SO_4$ 1N), 1h - en sachet.

Au cours du stade X, quatre doses d'enzyme ont été testées :

- X0 = abiotique ,
- X1 = 1 Unité enzymatique / g eq pâte sèche,
- X5 = 5 Unités enzymatiques / g eq pâte sèche,
- X10 = 10 Unités enzymatiques / g eq pâte sèche,

Après le stade X, la pâte a été essorée. Un aliquot de pâte a été prélevé pour la détermination de l'Indice Kappa (IK), la blancheur et la teneur en acides hexenuroniques (HexA).

Le stade chelatant (Q) a été suivi du stade PoP, qui a conduit à une pâte finale blanchie. A la fin de la séquence de blanchiment, la pâte a été lavée à l'eau du robinet, essorée et aliquotée. Des mesures de IK, blancheur, HexA, viscosité et caractéristiques physiques : longueur de rupture (LR, ISO 15361) et indice d'éclatement (IE, ISO 2758) ont été menées. L'effluent du stade P final a été collecté pour dosage du peroxyde résiduel. Un essai 'Contrôle' a également été réalisé selon une séquence normale (OO) QpoP.

## 2. Résultats

Ils sont donnés dans le tableau 4.

La pâte Kraft initiale (OO) présente un IK de 8,8 (i.e. ~1.3% de lignine) et une blancheur de 62% ISO (Tableau 3). La séquence de blanchiment doit permettre la dégradation et l'élimination des lignines résiduelles responsables de la couleur, et conduire à des pâtes ayant un IK plus faible, et une blancheur plus élevée.

L'étape de bio-traitement (X) est conduite à pH6 et à 50°C. Le bio-traitement entraine une baisse de l'IK des pâtes et une augmentation de la blancheur, plus marquée avec la dose enzymatique la plus forte (- 2,3 points d'IK et + 3,2 points de blancheur, X10) (Tableau 4). Le traitement abiotique (i.e. sans enzyme, X0) permet de réduire très légèrement l'IK de pâte (- 1,3 points) sans incidence toutefois sur la blancheur.

### Tableau 4 : Résultats des essais de Bio-Blanchiment

| Essais | | (OO) | (OO) X0 | (OO) X1 | (OO) X5 | (OO) X10 |
|---|---|---|---|---|---|---|
| Enzyme introduit U/g | | - | - | 1 | 5 | 10 |
| Activité Enz Effluent (U/ml) | Initial | | | 0.16 | 0.83 | 1.66 |
| Activité Enz Effluent (U/ml) | Final | | | 0.06 | 0.12 | 0.19 |
| pH final Effluent | | | 7.3 | 7.4 | 6.8 | 6.6 |
| IK | ± 5 % | 8.8 | 7.5 | 7.3 | 6.7 | 6.5 |
| Blancheur | ± 0.5 % | 62.3 | 62.2 | 63.2 | 64.8 | 65.5 |
| L* | | 90.33 | 90.29 | 90.52 | 90.95 | 91.08 |
| a* | | 0.07 | 0.01 | 0.06 | 0.04 | 0.05 |
| b* | | 12.91 | 12.98 | 12.41 | 11.69 | 11.35 |
| HexA (µmol/g) | ± 5 % | 82 | 77 | 77 | 73 | 69 |

| Essais | | (OO)QPoP | (OO) X0 QPoP | (OO) X1 QPoP | (OO) X5 QPoP | (OO) X10 QPoP |
|---|---|---|---|---|---|---|
| pH final Effluent | | 10.8 | 11 | 10.1 | 9.2 | 8.7 |
| H2O2 consommé % | | 2.99 | 2.98 | 2.96 | 2.96 | 2.95 |
| IK | ± 5 % | 5.8 | 5.3 | 4.8 | 4.5 | 4.3 |
| Blancheur | ± 0.5 % | 84 | 84.4 | 83.2 | 85.4 | 85.7 |
| L* | | 96.26 | 96.31 | 96.19 | 96.62 | 96.76 |
| a* | | -1.13 | -1.04 | -1.18 | -1.05 | -1.07 |
| b* | | 5.09 | 4.85 | 5.64 | 4.66 | 4.67 |
| HexA (µmol/g) | ± 5 % | 78 | 77 | 79 | 69 | 58 |
| Viscosité (ml/g) | ± 2 % | 753 | 788 | 804 | 830 | 738 |
| LR (km) | ± 4 % | 3.5 | 3.8 | 3.6 | 3.1 | 2.6 |
| Indice de Traction (N.m/g) | ± 4 % | 35 | 37 | 35 | 30 | 26 |
| Eclatement (kPa) | ±10 % | 140 | 150 | 137 | 113 | 92 |
| IE (kPa.m2/g) | ± 10 % | 1.7 | 1.8 | 1.7 | 1.4 | 1.2 |

L'évolution des paramètres L,a*,b* permet d'affiner les effets sur la 'blancheur' de la pâte. Il semble que le bio-traitement ait un effet plus marqué sur le facteur b* (-1,5 pts), ce qui indique une diminition de la contribution 'jaune'.

La pâte kraft délignifiée contient -80 µmol/g d'acides héxenuroniques (HexA). Ces acides proviennent de la dégradation des xylanes lors de la cuisson. Ces formes réductrices sont consommatrices de réactif de blanchiment et contribuent à l'IK.

L'intérêt des enzymes de type xylanase, est de :

- diminuer les teneurs en HexA,
- d'éliminer les xylanes déposés à la surface des fibres après cuisson,

- d'ouvrir les structures polymériques des fibres pour une meilleure diffusion des réactifs de blanchiment.

Le bio-traitement (stade X) permet de diminuer la teneur en HexA des pâtes, avec un effet plus marqué après les bio-traitements X5 et X10. Le traitement abiotique permet toutefois de réduire de 10 $\mu$mol/g la teneur en HexA, certainement engendré par la légère acidification de la pâte.

A la suite du stade de blanchiment QPoP, la pâte finale 'contrôle' présente une blancheur de 84% ISO (soit - + 22 pts), un IK de 5,8 (- 3 pts), et une teneur en HexA voisine de 80 $\mu$mol/g, soit relativement similaire à celle mesurée dans la pâte initiale (OO) (Tableau 4). Le traitement abiotique conduit à une pâte relativement similaire. Le pré-traitement par le cocktail enzymatique avant le stade QPoP, permet une amélioration significativeme de la blancheur et l'IK réduit les teneurs en HexA. Les effets les plus marqués sont observés avec la dose enzymatique la plus élevée (X10). Ainsi, la pâte finale (OO)X10 QPoP présente une blancheur élevée (+ 1,7 pts // contrôle), un IK plus faible (-1,5 pts // contrôle) ; et une teneur en HexA réduite (- 20 $\mu$mol/g // contrôle & abiotique).

Au cours du stade PoP, la consommation en $H_2O_2$ est très légèrement inférieure avec les pâtes bio-traitées. Compte tenus des conditions de blanchiment et du faible résiduel d'$H_2O_2$ (quantité consommée = quantité introduite), l'effet sur la quantité d'$H_2O_2$ consommée a certainement été nivelé.

Des caractéristiques physiques des pâtes finales ont également été déterminées telles que : viscosité, résistance à la traction (longueur de rupture, Indice de traction) et résistance à l'éclatement (éclatement, Indice d'éclatement) (Tableau 4). Le bio-traitement n'a pas d'effet significatif sur la viscosité de la pâte finale, au contraire le bio-traitement semble protéger la cellulose lors du blanchiment puisque les pâtes X0, X1 et X5 présentent une viscosité légèrement supérieure à celle de la pâte contrôle.

Les indice d'éclatement et indice de traction des pâtes finales contrôlées, abiotiques et X1, sont relativement similaires.

### 3. Conclusions

Ces essais montrent que le bio-traitement par la solution enzymatique permet d'améliorer significativement la qualité des pâtes avec une blancheur finale plus élevée (85,7 % ISO soit + 1,7 pts), un IK moindre (4,3, - 1,5 pts), avec une légère baisse de la consommation en $H_2O_2$. Les meilleurs résultats ont été observés avec une dose d'enzyme appliquée de 5 ou 10 U/ g de pâte eq. sèche. Une dose de 5 U/g permet d'obtenir des résultats intéressants (85,4 % ISO soit + 1,4 pts), un IK moindre (4,5 - 1,3 pts). Entre 1 et 5 U/g, les effets du bio-traitement ont augmenté avec la dose, alors qu'ils sont été nivelés pour des doses > 5 U/g.

Le bio-traitement à 5U/g conduit à une pâte dont les caractéristiques de résistances mécaniques sont similaires aux pâtes contrôle et abiotique.

En conclusion, le cocktail enzymatique appliqué au bio-blanchiment de pâte kraft d'eucalyptus, à une dose de 5 U/g, permet d'améliorer significativement le procédé de blanchiment, tout en limitant l'impact sur les caractéristiques mécaniques de la pâte.

### Exemple 6 : Cas du son de blé et de *Aspergilus niger.*

### Test en application : panification

Les essais ont été réalisés au Laboratoire-Fournil des Grands Moulins de Strasbourg, 1 place Henry Levy, 67016 Strasbourg.

### 1. Matériels et méthodes

#### 1.1 Enzymes :

Les enzymes (CAH) utilisées ont été produites comme indiqué dans l'exemple 3.

La référence utilisée par le Laboratoire-Fournil des Grands Moulins de Strasbourg est commercialisée par Danisco sous la référence Danisco Grindamyl 9001 (DG 9001). Il s'agit dune préparation d'enzyme connue et maîtrisée en panification, qui performe très bien sur de nombreux types de farines.

#### 1.2 Méthodes

#### *1.2.1* Mise en oeuvre

Les produits CAH et DG9001 sont testés en parallèle dans les pétrins 2 et 3. Le pétrin 1 n'est pas complémenté en enzyme et représente le témoin. A chacune des étapes les caractéristiques des pâtes sont notées.

**1.2.1.1 Ingrédients**

|  | **Pétrin 1** Témoin | **Pétrin 2** DG 9001 | **Pétrin 3** CAH |
|---|---|---|---|
| **Farine (base boulangère)** | 2 kg | 2 kg | 2kg |
| **Enzyme** | 0 | 300 mg (15 g/quintal) | 400 mg (20 g/quintal) |
| **Eau** | 1280 ml | 1280 ml | 1280 ml |
| **Levure** | 50 g | 50 g | 50 g |
| **Acide ascorbique** | 2g | 2g | 2g |
| **sel** | 44 g | 44 g | 44 g |

*1.2.1.2* **Pétrissage**

Le pétrissage est réalisé selon la séquence suivante :

- pétrissage 5 minutes à la première vitesse sur les pétrins à axe oblique VMI.
- pétrissage 15 minutes à la deuxième vitesse sur les pétrins à axe oblique VMI.
- ajout de 44 g de sel 5 minutes avant l'arrêt des pétrins.
- mesure de la température de la pâte.

*1.2.1.3* **Pointage**

- Fermentation

Les pâtes sont transférées des pétrins aux chambres de fermentation. Les conditions de la fermentation sont les suivantes : 27 °C, 20 min, hydrométrie 75 %.

- Division, boulage, détente
- Les pâtes sont sorties des chambres de fermentation. 6 pâtons de 350 g / pétrin sont boulés et laissés au repos pendant 20 minutes.

*1.2.1.4* **Façonnage**

Les pâtons sont façonnés mécaniquement (33 cm de long).

*1.2.1.5* **Fermentation**

La durée de fermentation n'est pas identique pour tous les pâtons. Pour chaque essai, 3 pâtons fermentent 2 h à 27°C, les trois autres fermentent 2h30 à 27°C (vérification de la tolérance de l'apprêt).

*1.2.1.6* **Cuisson**

Les pâtons façonnés issus des pétrins 1, 2, 3 sont scarifiés de 3 coups de lame et enfournés 25 minutes à 250 °C après introduction de vapeur d'eau.

**2. Résultats**

3. Le tableau 5 résume les résultats obtenus.

Le protocole utilisé s'appuie sur le test « BIPEA » devenu la norme AFNOR (NF V03 716) donnant une note globale de panification sur 300.

**Tableau 5**

| Pétrin | Temps de fermentation | Note pâte | Note pain | Volume | Valeur technologique | Note / 300 Valeur boulangère |
|---|---|---|---|---|---|---|
| **1** (Témoin) | 2:00h | 85 | 77,5 | 1650 | 162,5 | 262,5 |
| | 2 :30 h | 85 | 60,7 | 1690 | 145,7 | 245,7 |
| **2** (DG 9001) | 2:00h | 82 | 79,6 | 1720 | 161,6 | 261,6 |
| | 2 :30 h | 82 | 82 | 1700 | 164 | 264 |
| **3** (CAH) | 2:00h | 87 | 81,7 | 1690 | 168,7 | 268,7 |
| | 2 :30 h | 87 | 79 | 1800 | 166 | 266 |

**4. Conclusion**

Les résultats des tests en panification montrent que la qualité des pâtes ainsi que celle des pains obtenues avec le produit CAH sont supérieures à celles obtenues avec le produit DG9001. Les valeurs technologiques et boulangères du produit CAH mettent en évidence l'intérêt du produit CAH dans le domaine de la boulangerie par rapport à un produit commercial tel que le DG9001.

**Revendications**

**1.** Procédé de préparation de molécules d'intérêt par fermentation d'un substrat en phase solide comprenant les étapes suivantes :

a) prendre un substrat,

b) réduire éventuellement la taille dudit substrat de manière à obtenir des particules dont 100 % ont un diamètre moyen inférieur à 1 mm,

c) stériliser le substrat à sec,

d) incuber sous agitation le substrat obtenu à l'étape c) avec un microorganisme en suspension à raison de $1 \times 10^5$ à $1 \times 10^7$ spores/gramme de substrat, avantageusement $2,5 \times 10^5$ spores/gramme de substrat de manière à avoir un $a_w$ compris entre 0,950 et 0,999, avantageusement compris entre 0,980 et 0,995,

e) laisser fermenter à l'état solide dans un réacteur sans agitation pendant 2 à 8 jours, avantageusement 3 à 6 jours, à une température comprise entre 18 et 30 °C sous pression atmosphérique

f) stériliser le substrat fermenté obtenu à l'étape e),

g) reprendre le substrat obtenu à l'étape f) par un volume d'eau représentant 0,5 à 5 fois la masse de substrat,

h) recueillir le liquide de lavage,

i) extraire les molécules d'intérêt à partir du liquide recueilli à l'étape h) et optionnellement,

j) sécher les molécules d'intérêt recueillies à l'étape i).

**2.** Procédé selon la revendication 1 **caractérisé en ce que** l'étape c) est réalisée par irradiation.

**3.** Procédé selon la revendication 1 **caractérisé en ce que** l'étape f) est réalisée par irradiation antérieurement à l'extraction des molécules d'intérêt.

**4.** Procédé selon l'une quelconque des revendications 1 ou 2 **caractérisé en ce que** l'étape d) est réalisée en milieu aqueux.

**5.** Procédé selon l'une quelconque des revendications précédentes **caractérisé en ce que** le substrat est choisi dans le groupe comprenant la biomasse végétale, les matières premières agricoles, les sous-produits de l'industrie alimentaire et les boues d'épuration.

6.  Procédé selon l'une quelconque des revendications précédentes **caractérisé en ce que** le substrat est choisi dans le groupe comprenant les céréales comme par exemple le maïs, le blé, l'orge et le riz, le son de blé, la paille, les rafles de maïs, le bois de résineux ou de feuillu, les déchets végétaux, notamment les herbes et les feuilles, les déchets d'amidonnerie, la pulpe de betterave, le papier, la bagasse de canne à sucre, les résidus de papeterie.

7.  Procédé selon l'une quelconque des revendications précédentes **caractérisé en ce que** le microorganisme est choisi dans le groupe comprenant les bactéries, les champignons filamenteux ou non, en particulier les levures.

8.  Procédé selon la revendication 6 **caractérisé en ce que** le microorganisme utilisé est *Fusarium venenatum* déposé le 5 septembre 2001 sous le no : IMI 386918 ainsi *qu'Aspergillus niger* déposé le 5 mai 2011 sous le no IMI 500512 à CABI GRC (CAB International Genetic Resource Collection Nosworthy Way Wallingford Oxfordshire OX10 8DE Grande-Bretagne).

9.  Procédé selon l'une quelconque des revendications précédentes **caractérisé en ce que** les molécules d'intérêts obtenues sont choisies dans le groupe comprenant les cellulases, les xylanases, les $\alpha$-amylases, les arabinoxylanases, les $\alpha$-et $\beta$-glucosidases, les fucosidases, les protéases, les phytases, les phosphohydrolases, les phosphatases, les estérases, les glucosaminidases, les $\alpha$-et $\beta$-galactosidases, les $\alpha$-mannosidases et les lipases.

10. Procédé de préparation selon la revendication 1 **caractérisé en ce que** le substrat est le son de blé, le microorganisme est *Fusarium venenatum* déposé 5 septembre 2001 sous le no : IMI 386918 ainsi *qu'Aspergillus niger* déposé le 5 mai 2011 sous le no IMI 500512 à CABI GRC (CAB International Genetic Resource Collection Nosworthy Way Wallingford Oxfordshire OX10 8DE Grande-Bretagne) et les molécules d'intérêts celles de la revendication 9.

**FIGURE 1**

**FIGURE 2**

| | Europäisches Patentamt European Patent Office Office européen des brevets | **RAPPORT DE RECHERCHE EUROPEENNE** | Numéro de la demande EP 11 16 7963 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | RIGON SPIER M ET AL: "Phytase production using citric pulp and other residues of the agroindustry in SSF by fungal isolates.", FOOD TECHNOLOGY AND BIOTECHNOLOGY, vol. 46, no. 2, 1 avril 2008 (2008-04-01), pages 178-182, XP002615795, ISSN: 1330-9862 * abrégé; figures 1, 2, 4 * * page 179, colonne de gauche, alinéa 3 - colonne de droite, alinéa 2; tableau 1 * * page 181, colonne de droite, dernier alinéa * ----- | 1,2,4-7, 9 | INV. C12P21/00 C07K14/37 C12N9/14 C12N9/42 ADD. C12N1/14 C12R1/77 C12R1/685 |
| A | CEN PEILIN AND XIA LIMING: "Production of cellulase by solid-state fermentation", ADVANCES IN BIOCHEMICAL ENGINEERING, BIOTECHNOLOGY, SPRINGER, BERLIN, DE, vol. 65, 1 janvier 1999 (1999-01-01), pages 69-92, XP009107560, ISSN: 0724-6145, DOI: 10.1007/3-540-49194-5_4 * pages 72-80, alinéas 2.1-2.5, 3., 3.1-3.5 * ----- -/-- | 1-10 | DOMAINES TECHNIQUES RECHERCHES (IPC) C12P C12R C07K C12N |

Le présent rapport a été établi pour toutes les revendications

| Lieu de la recherche | Date d'achèvement de la recherche | Examinateur |
|---|---|---|
| Berlin | 23 septembre 2011 | Schröder, Gunnar |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 11 16 7963

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | SINGHANIA R R ET AL: "Advancement and comparative profiles in the production technologies using solid-state and submerged fermentation for microbial cellulases", ENZYME AND MICROBIAL TECHNOLOGY, STONEHAM, MA, US, vol. 46, no. 7, 31 mars 2010 (2010-03-31), pages 541-549, XP027038189, ISSN: 0141-0229 [extrait le 2010-05-01] * pages 541-542, alinéa 1. * * pages 544-545, alinéa 4. 4.1; tableau 3 * | 1-10 | |
| A,D | HATSCH DIDIER ET AL: "Development of a bipartite method for Fusarium identification based on cellobiohydrolase-C: CAPS and Western blot analysis", FEMS MICROBIOLOGY LETTERS, vol. 213, no. 2, 6 août 2002 (2002-08-06), pages 245-249, XP002615796, ISSN: 0378-1097 * abrégé; figure 3 * | 8,10 | |
| A | DATABASE MEDLINE [Online] US NATIONAL LIBRARY OF MEDICINE (NLM), BETHESDA, MD, US; mars 2010 (2010-03), ZHAO XUEBING ET AL: "Microwave pretreatment of substrates for cellulase production by solid-state fermentation.", XP002615840, Database accession no. NLM19452284 * abrégé *  -/-- | 2 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Berlin | 23 septembre 2011 | Schröder, Gunnar |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 11 16 7963

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| | & APPLIED BIOCHEMISTRY AND BIOTECHNOLOGY, vol. 160, no. 5, mars 2010 (2010-03), pages 1557-1571, ISSN: 1559-0291 ----- | | |
| A | DATABASE COMPENDEX [Online] ENGINEERING INFORMATION, INC., NEW YORK, NY, US; octobre 1993 (1993-10), KUME T ET AL: "Utilization of agro-resources by radiation treatment. Production of animal feed and mushroom from oil palm wastes", XP002615841, Database accession no. EIX93101053759 * abrégé * & RADIATION PHYSICS AND CHEMISTRY, vol. 42, no. 4-6 -6 pt 2, octobre 1993 (1993-10), pages 727-730, ----- | 2 | |
| A | PHALIP VINCENT ET AL: "Plant Cell Wall Degradation with a Powerful Fusarium graminearum Enzymatic Arsenal", JOURNAL OF MICROBIOLOGY AND BIOTECHNOLOGY, vol. 19, no. 6, juin 2009 (2009-06), pages 573-581 URL, XP002615860, ISSN: 1017-7825, DOI: 10.4014/jmb.0807.459 * abrégé; figure 1; tableau 1 * ----- -/-- | 8,10 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Berlin | 23 septembre 2011 | Schröder, Gunnar |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

& : membre de la même famille, document correspondant

EPO FORM 1503 03.82 (P04C02)

Europäisches
Patentamt

European
Patent Office

Office européen
des brevets

## RAPPORT DE RECHERCHE EUROPEENNE

Numéro de la demande

EP 11 16 7963

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | QIN YONGLING ET AL: "Isolation and characterization of a thermostable cellulase-producing Fusarium chlamydosporum", WORLD JOURNAL OF MICROBIOLOGY & BIOTECHNOLOGY, vol. 26, no. 11, 30 mars 2010 (2010-03-30) , pages 1991-1997, XP002615842, ISSN: 0959-3993 * abrégé * * page 1992, colonne de gauche, alinéa 2 * ----- | 8,10 | |
| X | US 2008/113414 A1 (BILANOVIC DRAGOLJUB D [US] ET AL) 15 mai 2008 (2008-05-15) * abrégé; figure 1 * * alinéas [0002], [0003], [0010] - [0024] * * exemples 1-6; tableaux 1-6 * ----- | 1,4-6 | |
| A | DATABASE WPI Week 200938 Thomson Scientific, London, GB; AN 2009-K05701 XP002659939, & CN 101 434 981 A (UNIV NANJING FINANCIAL) 20 mai 2009 (2009-05-20) * abrégé * ----- | 1-10 | DOMAINES TECHNIQUES RECHERCHES (IPC) |
| A | EP 1 022 329 A1 (GIE AGRO IND [FR]) 26 juillet 2000 (2000-07-26) * alinéas [0014] - [0029] * * exemples 2-6 * ----- | 1-10 | |

Le présent rapport a été établi pour toutes les revendications

| Lieu de la recherche | Date d'achèvement de la recherche | Examinateur |
|---|---|---|
| Berlin | 23 septembre 2011 | Schröder, Gunnar |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**   EP 11 16 7963

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-09-2011

| Document brevet cité au rapport de recherche | Date de publication | Membre(s) de la famille de brevet(s) | Date de publication |
|---|---|---|---|
| US 2008113414 A1 | 15-05-2008 | AUCUN | |
| CN 101434981 A | 20-05-2009 | AUCUN | |
| EP 1022329 A1 | 26-07-2000 | AT 294852 T | 15-05-2005 |
| | | AU 775240 B2 | 22-07-2004 |
| | | AU 3059800 A | 07-08-2000 |
| | | BR 0007600 A | 30-10-2001 |
| | | CA 2361264 A1 | 27-07-2000 |
| | | CN 1337998 A | 27-02-2002 |
| | | DE 60019825 D1 | 09-06-2005 |
| | | DE 60019825 T2 | 27-04-2006 |
| | | ES 2244395 T3 | 16-12-2005 |
| | | WO 0043496 A1 | 27-07-2000 |
| | | FR 2788782 A1 | 28-07-2000 |
| | | HU 0104907 A2 | 29-04-2002 |
| | | ID 29922 A | 25-10-2001 |
| | | JP 2002534973 A | 22-10-2002 |
| | | MA 25276 A1 | 01-10-2001 |
| | | MX PA01007528 A | 27-03-2003 |
| | | PL 350672 A1 | 27-01-2003 |
| | | PT 1022329 E | 31-08-2005 |
| | | TR 200102156 T2 | 21-05-2002 |
| | | US 2002037342 A1 | 28-03-2002 |
| | | ZA 200107009 A | 31-07-2002 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **HESSELTINE.** *International Biodeterioration,* 1987, vol. 23, 79-89
- **PANDEY et al.** Solid State Fermentation in Biotechnology - Fundamentals and Applications. Asiatech Publishers Inc, 2001
- **GODINEZ et al.** *Journal of Industrial Microbiology & Biotechnology,* 2001, vol. 26, 271-275
- **PAPAGIANNI et al.** *Process Biochemistry,* 1999, vol. 35, 397-402
- **PATIL ; DAYANAND.** *Bioresource Technology,* 2006, vol. 97, 2054-2058
- **SANDHYA et al.** *Process Biochemistry,* 2005, vol. 40, 2689-2694

- **VINIEGRA-GONZALEZ et al.** *Biochemical Engineering Journal,* 2003, vol. 13, 157-167
- **HATSCH et al.** Development of a bipartite method for Fusarium identification based on cellobiohydrolase-C : CAPS and Western blot analysis. *Fems Microbiology letters,* 2002, vol. 213, 245-249
- **STACK.** A Comparaison of the Inoculum Potential of Ascospores and Conidia of Giberella zeae. *Canadian Journal of Plant Pathology,* 1989, vol. 11, 137-142
- **BAILEY et al.** Interlaboratory testing of methods for assay of xylanase activity. *Journal of Biotechnology,* vol. 23 (3), 257-270